# EUROPEAN PATENT APPLICATION

(11) **EP 4 438 735 A1**
(43) Date of publication of application: **02.10.2024**
(21) Application number: 23164438.6
(22) Date of filing: 27.03.2023
(51) Int. Cl.: C12P 3/00, C12P 7/24, C12P 7/26, C12P 7/40, C12P 7/58, C12P 19/36

(54) **BIOCATALYTIC PREPARATION OF AN ORGANIC SUBSTANCE COMPRISING A CARBONYL GROUP**

(71) Applicant: Cascat GmbH, 94315 Straubing (DE)
(72) Inventor: SIEBER, Volker, 85405 Nandlstadt (DE); AL-SHAMERI, Ammar, 94315 Straubing (DE); PICK, André, 36179 Bebra-Breitenbach (DE)
(74) Representative: Aera A/S

(57) **Abstract**

The present invention relates to a process for preparing an organic substance comprising at least one carbonyl group comprising the steps of converting an organic substance comprising at least one oxygen-containing functional group into the organic substance comprising at least one carbonyl group and NAD+ and/or NADP+ into NADH and/or NADPH using a dehydrogenase, and converting NADH and/or NADPH into NAD+ and/or NADP+ and hydrogen using a hydrogenase.

## Description

The present invention relates to a process for preparing an organic substance comprising at least one carbonyl group, an organic substance comprising at least one carbonyl group obtainable by the process according to the invention, the use of a hydrogenase and a process for generating hydrogen in the oxidative production of an organic substance comprising at least one carbonyl group.

The long-term economic and environmental concerns associated with the petrochemical industry have provided the impetus for increased research, development, and commercialization of processes for conversion of carbon feedstocks into chemicals that can replace those petroleum feedstocks.

One approach is the development of biorefining processes to convert renewable feedstocks into products that can replace petroleum-derived chemicals.

Two common goals in improving a biorefining process include achieving a lower cost of production and reducing detrimental effects on the environment.

The biocatalytic production of most industrially important compounds involve oxidation-reduction (redox) reactions. Biosynthetic transformations involving redox reactions offer a significant economic and environmental advantage for the production of chemicals over conventional chemical processes, in particular those redox reactions requiring stereospecificity. Nicotinamide adenine dinucleotide (NAD) functions as a cofactor in over 300 redox reactions and regulates various enzymes and genetic processes. The NADH/NAD+ cofactor pair plays a major role in microbial catabolism.

Nicotinamide cofactor dependent enzymes are increasingly finding use for the synthesis of chemical compounds. Such processes are now in various stages of scale-up and commercialization. Biosynthetic transformations involving redox reactions also offer a considerable potential for the production of chemicals over conventional chemical processes. Enzymes referred to in general as oxidoreductases, or more specifically as oxidases, reductases or dehydrogenases, catalyze these biological redox reactions. These enzymes require a donor and/or an acceptor of reducing equivalents in the form of electrons, hydrogen or oxygen atoms. Cofactor pairs that are transformed reversibly between their reduced and oxidized states, nucleotide cofactors such as NADH/NAD+ and NADPH/NADP+ among others, serve as donors and/or acceptors of reducing equivalents very effectively in a living cell.

Because of the relatively high cost of nicotinamide cofactors (in comparison to the other starting materials), it is not economically feasible to use the cofactor in stoichiometric quantities. The effective regeneration of used cofactors is critical in industrial cofactor-dependent production systems because of the impeding high cost of cofactors such as NAD. The cofactors, also referred to as co-enzymes, NADH/NAD+ and NADPH/NADP+ are expensive chemicals, thereby making their regeneration by reduction/oxidation to the original state imperative if they are to be used economically in low cost, chemical production systems. It would therefore be desirable if the cofactor could be regenerated in situ using a suitable recycling system.

Efforts to do so have been described in US4,766,071, describing in vitro regeneration of NADH using a cell lysate of *Clostriduim kluyveri* as a biocatalyst and an aldehyde as an oxidizing agent. US5,302,520 discloses a NAD regeneration system and an adenosine phosphate regeneration system that, in the presence of pyruvate, yields a labeled carbohydrate. These methods only focus on the regeneration of the reduced form of the cofactor, i.e. NADH/NADPH and not of the oxidized form, i.e. NAD+/NADP+.

The production of chemicals by oxidation requires the simultaneous reduction of the cofactor and its recycling in the further process. This requires co-substrates, which make the process more complex and the reprocessing more difficult. One possibility here is the use of NADH oxidases. These require oxygen and produce water or hydrogen peroxide from it; in the case of hydrogen peroxide, a catalase is also required. Due to the low solubility of oxygen in aqueous solutions, a constant supply is required. However, this gassing leads to problems with the stability of the enzymes, which severely limits these systems. For this reason, alternative systems for recycling the oxidized cofactor are urgently needed.

Aiming at the problems that the existing cofactor regeneration systems are strongly limited to the regeneration of the reduced cofactors, the present invention provides a process aiming at the regeneration of the oxidized cofactors for improved conversion. In enzyme bioreactors, NAD+-dependent formate dehydrogenase (FDH) is used to regenerate NADH from NAD+ in vitro. FDH catalyzes the practically irreversible oxidation of formate to CO₂ and the simultaneous reduction of NAD+ to NADH. This system of cofactor regeneration has been successfully applied in the production of optically active amino acids as well as chiral hydroxy acids, esters, alcohols, and other fine chemicals synthesized by different dehydrogenases. A process which also provides a gaseous by-product during the regeneration of NAD+/NADP+ does not exist.

It is the object of the present invention to provide a process for the preparation of organic substances with an efficient recycling of the cofactors NAD+/NADP+ and NADH/NADPH.

This object is solved by the process for preparing an organic substance comprising at least one carbonyl group, an organic substance comprising at least one carbonyl group obtainable by the process according to the invention, the use of a hydrogenase for recycling NADH to NAD+ and/or NADPH to NADP+ in a process applying a dehydrogenase and an organic substance, the use of a hydrogenase for generating hydrogen in a process using a dehydrogenase, the use of a hydrogenase in a process for preparing an organic substance comprising at least one carbonyl group applying a dehydrogenase and NAD+/NADH and/or NADP+/NADPH, for generating hydrogen and NAD+ and/or NADP+, and a process for generating hydrogen in the oxidative production of an organic substance comprising at least one carbonyl group.

In a first aspect the invention relates to a process for preparing an organic substance comprising at least one carbonyl group comprising, preferably consisting of, the steps of converting an organic substance comprising at least one oxygen-containing functional group into the organic substance comprising at least one carbonyl group and NAD+ and/or NADP+ into NADH and/or NADPH using a dehydrogenase, and converting NADH and/or NADPH into NAD+ and/or NADP+ and hydrogen using a hydrogenase.

It has been surprisingly found that the inventive process allows the efficient recycling of the cofactor NAD(P)H / NAD(P)+. The hydrogenase which converts NAD(P)H into NAD(P)+ also produces hydrogen as a by-product. By outgassing the produced hydrogen, a constant shift of the equilibrium in favor of the products in the process can be achieved.

This finding is even more surprising as the oxidation of NADH/NADPH with concomitant formation of hydrogen is expected to barely taking place as soon as some hydrogen is formed the back reaction, that is, the reduction of NAD+/NADP+ will occur. Surprisingly it was found that the production of hydrogen will proceed and full conversion in the recycling of NAD+/NADP+ can be achieved.

By chance the use of a hydrogenase proved to be a superior approach for recycling in comparison to approaches know in the art which require NADH oxidases due to several factors. Bubbling of oxygen or more general a gas into the aqueous production system becomes superfluous. This results in much less destabilization of the used biocatalysts. Instead, a gas (hydrogen) is generated, which can be easily removed and this allows the constant shift to the product.

Furthermore, the present invention provides coupled systems for the efficient biosynthesis of different organic molecules coupled with a cofactor regenerating system comprising a hydrogenase.

While the present disclosure describes aspects and specific embodiments, those skilled in the art will recognize that various changes may be made and equivalents may be substituted without departing from this disclosure. The present disclosure is not limited to particular nucleic acids, expression vectors, enzymes, biosynthetic pathways, host microorganisms, or processes, as such may vary. The terminology used herein is for the purposes of describing particular aspects and embodiments only, and is not to be construed as limiting. In addition, many modifications may be made to adapt a particular situation, material, composition of matter, process, process steps or process flows, in accordance with this disclosure. All such modifications are within the scope of the claims appended hereto.

The redox cofactor nicotinamide adenine dinucleotide, NAD, comes in two forms - phosphorylated and un-phosphorylated. The term NAD(P) refers to both phosphorylated (NADP) and un-phosphorylated (NAD) forms, and encompasses oxidized versions (NAD+ and NADP+) and reduced versions (NADH and NADPH) of both forms. The term "NAD(P)+" refers to the oxidized versions of phosphorylated and un-phosphorylated NAD, i.e., NAD+ and NADP+. Similarly, the term "NAD(P)H" refers to the reduced versions of phosphorylated and un-phosphorylated NAD+, i.e., NADH and NADPH. When NAD(P)H is used to describe the redox cofactor in an enzyme catalyzed reaction, it indicates that NADH and/or NADPH is used. Similarly, when NAD(P)+ is the notation used, it indicates that NAD+ and/or NADP+ is used. While many proteins may bind either a phosphorylated or un-phosphorylated cofactor, there are redox cofactor promiscuous proteins, natural or engineered, that are indiscriminate; in these cases, the protein may use either NADH and/or NADPH. In some embodiments, enzymes that preferentially utilize either NAD(P) or NAD may carry out the same catalytic reaction when bound to either form.

It is to be understood that a carbonyl group is a functional group with the formula C=O, composed of a carbon atom double-bonded to an oxygen atom, and it is divalent at the C atom. It is known to the person skilled in the art that a compound containing a carbonyl group is often referred to as a carbonyl compound. Carbonyl compounds containing a carbonyl group are aldehyde, ketone, carboxylic acid, carboxylate ester, amide, enone, acyl halide, acid anhydride, imide, urea, thioester and the like. It is to be understood that respective salts are encompassed.

The classification or discussion of a material in any section of this specification as having a particular utility (e.g., "catalyst") is for convenience.

The citation of references herein does not constitute an admission that such references are prior art or relevant to the patentability of the invention disclosed herein. Any discussion of the contents of the references cited is intended to provide only a general summary of the claims made by the authors of the references and does not constitute an admission as to the correctness of the contents of such references.

While the description and specific examples provide embodiments of the invention, they are for illustrative purposes only and are not intended to limit the scope of the invention. Furthermore, mention of several embodiments having the features mentioned is not intended to exclude other embodiments having additional features or other embodiments having other combinations of the features mentioned. Specific examples are provided to illustrate how the compositions and methods of the present invention may be made and used and, unless otherwise expressly stated, are not to be construed as representations that particular embodiments of the present invention have or have not been made or tested.

As used herein, the words "preferred" and "preferably" refer to embodiments of the invention that provide certain advantages under certain circumstances. However, other embodiments may also be preferred under the same or different circumstances. Moreover, mention of one or more preferred embodiments does not imply that other embodiments are not useful and is not intended to exclude other embodiments from the scope of the invention.

According to a preferred embodiment the oxygen-containing functional group is selected from the group consisting of hydroxyl, ketone, aldehyde, ester, amid, ether, semi-acetal or acetal, preferably is a hydroxyl group, an aldehyde group or a ketone group, more preferably is a hydroxyl group or aldehyde group, and even more preferably is a hydroxyl group.

It is further preferred that the carbonyl group of the organic substance comprising at least one carbonyl group is selected from the group consisting of aldehyde group, ketone group or carboxyl group.

It is to be understood that in the inventive process for converting an organic substance comprising at least one oxygen-containing functional group into the organic substance comprising at least one carbonyl group that this step comprises an oxidation. It is to be understood that the oxygen-containing functional group is converted into a carbonyl group, e.g. a hydroxyl group is converted into an aldehyde or an aldehyde is converted into a carboxylic acid.

Under hydrogenase any enzyme falling within the classification EC 1.12.X.X or more specific EC 1.12.99.X is to be understood.

According to a preferred embodiment the hydrogenase is selected from the group consisting of Ralstonia eutropha (SH) and/or SHE341A/S342R, preferably is Ralstonia eutropha (SH); and/or an oxidoreductase is not applied in the process.

Preferably the hydrogenase tolerates oxygen or even more preferably the hydrogenase is an oxygen stabile hydrogenase.

Under dehydrogenase any enzyme falling within the classification EC 1.1.1.X is to be understood. According to a preferred embodiment the dehydrogenase may be selected from the group consisting of EC1.1.1.1 alcohol dehydrogenase, EC 1.1.1.2 alcohol dehydrogenase (NADP(+)), EC 1.1.1.47 glucose 1-dehydrogenase [NAD(P)(+)], EC 1.1.1.48 D-galactose 1-dehydrogenase, EC 1.1.1.49 glucose-6-phosphate dehydrogenase (NADP(+)), EC 1.1.1.67 mannitol 2-dehydrogenase, EC 1.1.1.71 alcohol dehydrogenase [NAD(P)(+)], EC 1.1.1.113 L-xylose 1-dehydrogenase, EC 1.1.1.114 apiose 1-reductase, EC 1.1.1.115 ribose 1-dehydrogenase (NADP(+)), EC 1.1.1.116 D-arabinose 1-dehydrogenase (NAD(+)), EC 1.1.1.117 D-arabinose 1-dehydrogenase [NAD(P)(+)], EC 1.1.1.118 glucose 1-dehydrogenase (NAD(+)), EC 1.1.1.119 glucose 1-dehydrogenase (NADP(+)), EC 1.1.1.120 galactose 1-dehydrogenase (NADP(+)), EC 1.1.1.121 aldose 1-dehydrogenase (NAD(+)), EC 1.1.1.173 L-rhamnose 1-dehydrogenase, EC 1.1.1.175 D-xylose 1-dehydrogenase, EC 1.1.1.179 D-xylose 1-dehydrogenase (NADP(+), D-xylono-1,5-lactone-forming), EC 1.1.1.203 uronate dehydrogenase, EC 1.1.1.250 D-arabinitol 2-dehydrogenase, EC 1.1.1.251 galactitol-1-phosphate 5-dehydrogenase, EC 1.1.1.255 mannitol dehydrogenase, EC 1.1.1.316 L-galactose 1-dehydrogenase, 1.1.1.359 aldose 1-dehydrogenase [NAD(P)(+)], 1.1.1.360 glucose/galactose 1-dehydrogenase, EC 1.1.1.376 L-arabinose 1-dehydrogenase [NAD(P)(+)], EC 1.1.1.377 L-rhamnose 1-dehydrogenase (NADP(+)), EC 1.1.1.378 L-rhamnose 1-dehydrogenase [NAD(P)(+)], EC 1.1.1.388 glucose-6-phosphate dehydrogenase (NAD(+)), EC 1.1.1.389 2-dehydro-3-deoxy-L-galactonate 5-dehydrogenase, EC 1.1.1.406 galactitol 2-dehydrogenase (L-tagatose-forming), or EC 1.1.1.407 D-altritol 5-dehydrogenase.

According to another preferred embodiment the process is a one-pot reaction.

An advantage of this process is that it performs all the catalytic steps in the same reaction batch without the need to isolate intermediates neither to add enzymes or substrates for cofactor recycling. The process can be operated either batchwise or continuously.

Further preferred the process is 100% atom efficient.

In a further preferred embodiment NAD+/NADH and/or NADP+/NADPH is applied in non-stochiometric quantities.

According to another preferred embodiment the yield of the conversion from NADH to NAD+ is more than 75%, preferably more than 80% and more preferably more than 90%.

It is further preferred that no sacrificial substrate is necessary, preferably no sacrificial substrate for NAD+/NADH and/or NADP+/NADPH recycling is necessary.

This has the advantage that less reactants are necessary which reduces the costs of the process and also simplifies the work up and purification.

It is further preferred that the process does not need the addition of O₂, preferably does not need the addition of O₂ for NAD+/NADH and/or NADP+/NADPH recycling.

It is preferred that the process is run at a temperature in the range of from 5 to 100°C, preferably of from 15 to 60°C, and more preferably of from 20° to 40°C.

It is preferred that the pH in the inventive process is between 3 to 12, and preferably between 4 to 10.

It is also possible to supplement the aqueous system by a buffer system. Suitable buffers (systems) are known and include conventional buffers (systems), for example, acetate, potassium phosphate, Tris-HCl, glycylglycine and glycine buffers, or mixtures of these. Preferably, a buffer used in a method of the present invention has a pH of 3 to 12, preferably of 4 to 12, more preferably of 4 to 11. For optimum activity of the biocatalysts ions, e.g. Mg²⁺, may be added. The use of stabilizers, glycerol etc. may allow longer use of biocatalysts.

According to another preferred embodiment the oxidized organic substance comprising at least one carbonyl group is converted further using further enzymes; and/or at least one further enzyme is used.

Enzymes suitable for the further conversion of the organic substance comprising at least one carbonyl group may be selected from the group consisting of lactonase (EC 3.1.1.15, EC 3.1.1.17, EC 3.1.1.14, EC 3.1.1.25), decarboxylase (EC 4.1.1.1, EC 4.1.1.2, EC 4.1.1.4, EC 4.1.1.5, EC 4.1.1.7, EC 4.1.1.40, EC 4.1.1.43), aldolase (EC 4.1.2.28, EC 4.1.2.29, EC 4.1.2.51, EC 4.1.2.52, EC 4.1.2.53, EC 4.1.2.54, EC 4.1.2.55, EC 4.1.3.16, EC 4.1.3.17, EC 4.1.3.39, EC 4.1.3.42, EC 4.1.3.43), dehydratase (EC 4.2.1.5, EC 4.2.1.6, EC 4.2.1.7, EC 4.2.1.8, EC 4.2.1.9, EC 4.2.1.12, EC 4.2.1.25, EC 4.2.1.28, EC 4.2.1.30, EC 4.2.1.32, EC 4.2.1.34, EC 4.2.1.35, EC 4.2.1.39, EC 4.2.1.40, EC 4.2.1.41, EC 4.2.1.42, EC 4.2.1.43, EC 4.2.1.67, EC 4.2.1.68, EC 4.2.1.81, EC 4.2.1.82, EC 4.2.1.90, EC 4.2.1.140, EC 4.2.1.141, EC 4.2.1.146, EC 4.2.1.156, EC 4.2.1.158, EC 4.2.1.162, EC 4.2.1.176, EC 4.3.1.9).

Preferably the conversion of the organic substance comprising at least one oxygen-containing functional group to obtain the organic substance comprising at least one carbonyl group is part of a multi-enzymatic cascade.

According to a preferred embodiment the organic substance comprising at least one oxygen-containing functional group is a carbohydrate; or
the organic substance comprising at least one oxygen-containing functional group, preferably at least one hydroxyl group or aldehyde group, comprises at least one additional oxygen-containing functional group, preferably at least one additional hydroxyl group; and/or
wherein the organic substance comprising at least one oxygen-containing functional group is a polyol.

Further preferred the organic substance comprising at least one oxygen-containing functional group is an aldose, preferably the aldose is converted to an aldonic acid or a salt thereof; or
the organic substance comprising at least one oxygen-containing functional group is a C6 or C5 carbohydrate being converted to α-keto-glutarate; or
the organic substance comprising at least one oxygen-containing functional group is a C6 or C5 carbohydrate being converted to succinic acid or a salt thereof; or
the organic substance comprising at least one oxygen-containing functional group is a C6 carbohydrate being converted to pyruvate; or
the organic substance comprising at least one oxygen-containing functional group is a polyol being converted to an aldose; or
the organic substance comprising at least one oxygen-containing functional group is a furfural or hydroxymethyl furfural being converted to a carboxylic acid or dicarboxylic acid.

In a further preferred embodiment the hydrogen generated during the process is removed from the process. This has the advantage that the equilibrium is favourably shifted.

Further preferred the process is not sensitive to O₂; and/or the process is a cell-free process.

Preferably the process is a circular process; and/or the process is a continuous process; and/or the NAD+/NADH and/or NADP+/NADPH is recycled in the process.

In a second aspect the present invention relates to an organic substance comprising at least one carbonyl group obtainable by the process according to the invention.

All remarks with respect to the process for preparing an organic substance comprising at least one carbonyl group as mentioned hereinbefore also apply also to this aspect where applicable.

In a third aspect the invention relates to the use of a hydrogenase for recycling NADH to NAD+ and/or NADPH to NADP+ in a process applying a dehydrogenase and an organic substance with at least one oxygen-containing functional group.

All remarks with respect to the process for preparing an organic substance comprising at least one carbonyl group as mentioned hereinbefore also apply also to this aspect where applicable. Hydrogen is generated in the recycling process.

In a fourth aspect the invention relates to the use of a hydrogenase for generating hydrogen in a process using a dehydrogenase, an organic substance comprising at least one oxygen-containing functional group and NAD+/NADH and/or NADP+/NADPH.

All remarks with respect to the process for preparing an organic substance comprising at least one carbonyl group as mentioned hereinbefore also apply also to this aspect where applicable.

In a fifth aspect the invention relates to the use of a hydrogenase in a process for preparing an organic substance comprising at least one carbonyl group applying a dehydrogenase and NADH and/or NADPH, for generating hydrogen and NAD+ and/or NADP+.

All remarks with respect to the process for preparing an organic substance comprising at least one carbonyl group as mentioned hereinbefore also apply also to this aspect where applicable.

In a sixth aspect the present invention relates to a process for generating hydrogen in the oxidative production of an organic substance comprising at least one carbonyl group, preferably comprising at least one carboxyl group, aldehyde group or ketone group, wherein the reactant being oxidized is an organic substance comprising at least one oxygen-containing functional group, wherein the oxygen-containing functional group is selected from the group of functional groups consisting of hydroxyl, ketone, aldehyde, ester, amid, ether, semi-acetal or acetal, preferably is a hydroxyl group, an aldehyde group or a ketone group, more preferably is a hydroxyl group or an aldehyde group, even more preferably is a hydroxyl group.

All remarks with respect to the process for preparing an organic substance comprising at least one carbonyl group as mentioned hereinbefore also apply also to this aspect where applicable.

While the invention has been described with reference to certain preferred embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiments, but that the invention will include all embodiments falling within the scope of the appended claims.

### Abbreviations

PuDHT - *Paralcaligenes ureilyticus* DSM 24591 dihydroxy acid dehydratase (SEQ ID NO: 7, SEQ ID NO: 8)
EcAdhZ3-LND - *Escherichia coli* alcohol dehydrogenase adhZ3 variant LND (SEQ ID NO: 11, SEQ ID NO: 12)
HsXylDHI - *Herbaspirillum seropedicae* DSM 6445 xylose Dehydrogenase I (SEQ ID NO: 1, SEQ ID NO: 2)
HsXyIDHII- *Herbaspirillum seropedicae* DSM 6445 xylose Dehydrogenase II (SEQ ID NO: 3, SEQ ID NO: 4)
NmLacll - *Noviherbaspirillum massiliense* JC206 DSM25712 lactonase II (SEQ ID NO: 5, SEQ ID NO: 6)
BsGDH E170KQ252L - Bacillus subtilis glucose dehydrogenase variant (SEQ ID NO: 13, SEQ ID NO: 14)
BsGDH P45AN46EF155YE170KV227AW230FQ252L - Bacillus subtilis glucose dehydrogenase variant (SEQ ID NO: 15, SEQ ID NO: 16)
AtUDH - Agrobacterium tumefaciens C58 uronate dehydrogenase (SEQ ID NO: 17, SEQ ID NO: 18)
AsGlucD -Actinobacillus succinogenes 130Z glucarate dehydratase (SEQ ID NO: 19, SEQ ID NO: 20)
AbKdgD - Acinetobacter baylyi ADP1 keto-deoxy glucarate dehydratase (SEQ ID NO: 21, SEQ ID NO: 22)
PpKgsaDH - Pseudomonas putida DSM 6125 alpha-ketoglutarate semialdehyde dehydrogenase (SEQ ID NO: 29, SEQ ID NO: 30)
PpDKdgD - Pseudomonas putida DSM 6125 D-keto-deoxy-pentanoate dehydratase (SEQ ID NO: 25, SEQ ID NO: 26)
CnLKdgD - Cupriavidus necator DSM 13513 L-keto-deoxy-pentanoate dehydratase (SEQ ID NO: 27, SEQ ID NO: 28)
PtKdgA- Picrophilus torridus keto-deoxy gluconate aldolase (SEQ ID NO: 31, SEQ ID NO: 32)
LIKdcA - *Lactococcus lactis* branched chain keto-acid decarboxylase (SEQ ID NO: 9, SEQ ID NO: 10)
SspADH- Sphingomonas sp. alcohol dehydrogenase (SEQ ID NO: 33, SEQ ID NO: 34)
MtKgd - Mycobacterium tuberculosis H37Rv keto-glutarate decarboxylase (SEQ ID NO: 35, SEQ ID NO: 36)
ApPDC - Acetobacter pasteurianus pyruvate decarboxylase (SEQ ID NO: 39, SEQ ID NO: 40)
RsGatDH- Rhodobacter sphaeroides D galactitol dehydrogenase (SEQ ID NO: 37, SEQ ID NO: 38)
VpAlda - Variovorax paradoxus aldehyde dehydrogenase (SEQ ID NO: 23, SEQ ID NO: 24)
ReSH - Ralstonia eutropha soluble hydrogenase

### EXPERIMENTAL SECTION

### Materials and Methods

D-Glucose, D-Mannose, D-Galactose, D-Xylose, L-Arabinose, L-Gulose, Sodium-D-glucuronate, D-Galacturonic acid sodium salt, D-Sorbitol, Dulcitol, 5-hydroxymethylfurfural (HMF), 2,5-Furandicarboxylic acid (FDCA) were obtained commercially from Carbosynth.

### Enzyme production and purification

In brief, a plasmid encoding respective enzyme was used to transform *E. coli* BL21 (DE3).

*Hs*XylDHI (SEQ ID NO: 1), *Hs*XyIDHII (SEQ ID NO: 3) and NmLacll (SEQ ID NO: 5) were expressed in autoinduction media at 37 °C for 3 h. before shifting the temperature to 16 °C for 16 h (Sutiono, S., Pick, A., & Sieber, V.. Converging conversion-using promiscuous biocatalysts for the cell-free synthesis of chemicals from heterogeneous biomass. Green Chemistry 2021. 23 (10), 3656-3663. doi.org/10.1039/D0GC04288A). Cell pellet was harvested and kept at -80 °C prior to purification.

*Pu*DHT (SEQ ID NO: 7) was expressed in TB media and induced using IPTG as described previously (Sutiono. S.; Teshima. M.; Beer. B.; Schenk. G.; Sieber. V. Enabling the Direct Enzymatic Dehydration of D-Glycerate to Pyruvate as the Key Step in Synthetic Enzyme Cascades Used in the Cell-Free Production of Fine Chemicals. ACS Catal. 2020. 10 (5). 3110-3118. doi.org/10.1021/acscatal.9b05068).

*Ll*KdcA (SEQ ID NO: 9) was expressed in autoinduction media at 37 °C for 3 h. before shifting the temperature to 25 °C for 16 h (Sutiono, S., Satzinger, K., Pick, A., Carsten, J., & Sieber, V. To beat the heat-engineering of the most thermostable pyruvate decarboxylase to date. RSC advances, 2019 9(51), 29743-29746. https://doi.org/10.1039/C9RA06251C). Cell pellet was harvested and kept at -80 °C prior to purification.

EcAdhZ3-LND (SEQ ID NO: 11) was expressed in autoinduction media containing 0.1 mM ZnCl₂ at 37 °C for 3 h. before shifting the temperature to 16 °C for 16 h (Pick. A.; Rühmann. B.; Schmid. J.; Sieber. V. Novel CAD-like Enzymes from Escherichia Coli K-12 as Additional Tools in Chemical Production. Appl. Microbiol. Biotechnol. 2013. 97 (13). 5815-5824. https://doi.org/10.1007/s00253-012-4474-5; Pick, A., Ott, W., Howe, T., Schmid, J., & Sieber, V.. Improving the NADH-cofactor specificity of the highly active AdhZ3 and AdhZ2 from Escherichia coli K-12. Journal of Biotechnology, 2014 189, 157-165. https://doi.org/10.1016/j.jbiotec.2014.06.015). Cell pellet was harvested and kept at -80 °C prior to purification.

BsGDH E170KQ252L (SEQ ID NO: 13) and BsGDH P45AN46EF155YE170KV227AW230FQ252L (SEQ ID NO: 15) were expressed in autoinduction media at 37 °C for 3 h before shifting the temperature to 16 °C for 16 h (Vázquez-Figueroa, E., Chaparro-Riggers J., Bommarius A.S.. Development of a thermostable glucose dehydrogenase by a structure-guided consensus concept." ChemBioChem 2007 8 (18): 2295-2301. doi.org/10.1002/cbic.200700500). Cell pellet was harvested and kept at -80 °C prior to purification.

AtUDH (SEQ ID NO: 17) was expressed in autoinduction media at 37 °C for 3 h. before shifting the temperature to 25 °C for 16 h (Pick, A., Schmid, J., & Sieber, V. Characterization of uronate dehydrogenases catalysing the initial step in an oxidative pathway. Microbial biotechnology, 2015 8(4), 633-643. doi.org/10.1111/1751-7915.12265). Cell pellet was harvested and kept at -80 °C prior to purification.

AsGlucD (SEQ ID NO: 19) was expressed in autoinduction media at 37 °C for 3 h. before shifting the temperature to 25 °C for 16 h (Aghaie, A., Lechaplais, C., Sirven, P., Tricot, S., Besnard-Gonnet, M., Muselet, D., Perret, A. New insights into the alternative D-glucarate degradation pathway. Journal of Biological Chemistry, 2008 283(23), 15638-15646 doi.org/10.1074/jbc.M800487200). Cell pellet was harvested and kept at -80 °C prior to purification.

AbKdgD (SEQ ID NO: 21) was expressed in TB media supplemented with 1 M sorbitol and 5 mM betaine at 37 °C for 3 h. before shifting the temperature to 25 °C for 16 h (Pick A, Beer B, Hemmi R, Momma R, Schmid J, Miyamoto K, Sieber V Identification and characterization of two new 5-keto-4-deoxy-D-Glucarate Dehydratases/Decarboxylases. BMC Biotechnology, 2016 16(1): p. 80.doi: 10.1186/s12896-016-0308-3). Cell pellet was harvested and kept at -80 °C prior to purification.

VpAldA (SEQ ID NO: 23) was expressed in autoinduction media at 37 °C for 3 h. before shifting the temperature to 16 °C for 16 h. Cell pellet was harvested and kept at -80 °C prior to purification.

PpDKdpD (SEQ ID NO: 25) and CnLKdpD (SEQ ID NO: 27) were expressed in autoinduction media at 37 °C for 3 h. before shifting the temperature to 30 °C for 16 h (Sutiono, S.; Siebers, B.; Sieber, V., Characterization of highly active 2-keto-3-deoxy-Larabinonate and 2-keto-3-deoxy-D-xylonate dehydratases in terms of the biotransformation of hemicellulose sugars to chemicals. Appl. Microbiol. Biotechnol. 104 (16), (2020) 7023-7035. doi.10.1007/s00253-020-10742-5). Cell pellet was harvested and kept at -80 °C prior to purification.

PpKgsaDH (SEQ ID NO: 29) was expressed in autoinduction media at 37 °C for 3 h. before shifting the temperature to 16 °C for 16 h (Sutiono, S., Pick, A., Sieber, V. Converging conversion - using promiscuous biocatalysts for the cell-free synthesis of chemicals from heterogeneous biomass. Green Chemistry. 2021 23. 10.1039/D0GC04288A.). Cell pellet was harvested and kept at -80 °C prior to purification.

PtKdgA (SEQ ID NO: 31) was expressed in autoinduction media at 37 °C for 3 h. before shifting the temperature to 25 °C for 16 h (Gmelch, T. J., Sperl, J. M., & Sieber, V.. Optimization of a reduced enzymatic reaction cascade for the production of L-alanine. Scientific reports 2019, 9(1), 11754. doi: 10.1038/s41598-019-48151-y). Cell pellet was harvested and kept at -80 °C prior to purification.

SspADH (SEQ ID NO: 33) was expressed in autoinduction media at 37 °C for 3 h. before shifting the temperature to 16 °C for 16 h (Beer B, Pick A, Döring M, Lommes P, Sieber V. Substrate scope of a dehydrogenase from Sphingomonas species A1 and its potential application in the synthesis of rare sugars and sugar derivatives. Microbial Biotechnology 2018 11(4), 747-758 doi:10.1111/1751-7915.13272.). Cell pellet was harvested and kept at -80 °C prior to purification.

MtKgd ((SEQ ID NO: 35) was expressed as described in the literature (Wagner, T., Bellinzoni, M., Wehenkel, A., O'Hare, H. M., & Alzari, P. M. (2011). Functional plasticity and allosteric regulation of α-ketoglutarate decarboxylase in central mycobacterial metabolism. Chemistry & biology, 18(8), 1011-1020. doi.org/10.1016/j.chembiol.2011.06.004). Cell pellet was harvested and kept at -80 °C prior to purification.

RsGatDH (SEQ ID NO. 37) was expressed as described in the literature (Demir, A. S., Talpur, F. N., Sopaci, S. B., Kohring, G. W., & Celik, A. Selective oxidation and reduction reactions with cofactor regeneration mediated by galactitol-, lactate-, and formate dehydrogenases immobilized on magnetic nanoparticles. Journal of biotechnology, 2011 152(4), 176-183. doi.org/10.1016/j.jbiotec.2011.03.002). Cell pellet was harvested and kept at -80 °C prior to purification.

ApPDC (SEQ ID NO. 39) was expressed as described in the literature (Sutiono, S., Satzinger, K., Pick, A., Carsten, J., & Sieber, V. To beat the heat-engineering of the most thermostable pyruvate decarboxylase to date. RSC advances, 2019 9(51), 29743-29746. doi.org/10.1039/C9RA06251C). Cell pellet was harvested and kept at -80 °C prior to purification. ReSH was expressed like described in the literature (Schiffels, J., Pinkenburg, O., Schelden, M., Aboulnaga, E. H. A., Baumann, M. E., & Selmer, T. An innovative cloning platform enables large-scale production and maturation of an oxygen-tolerant [NiFe]-hydrogenase from Cupriavidus necator in Escherichia coli. PloS one, 2013 8(7), e68812. doi.org/10.1371/journal.pone.0068812). Also the variant ReSH E341A/S342R was generated (Preissler, J., Reeve, H. A., Zhu, T., Nicholson, J., Urata, K., Lauterbach, L., & Lenz, O.. Dihydrogen-Driven NADPH Recycling in Imine Reduction and P450-Catalyzed Oxidations Mediated by an Engineered O2-Tolerant Hydrogenase. ChemCatChem, 2020 12(19), 4853-4861.) to allow also the phosphorylated from of the cofactor (NADP⁺/NADPH) to be used.

All enzymes expressed harboring hexahistidine in their N-terminal or C-terminal. For purification the cell pellet was first disrupted using sonicator at 80% and 0.5 s cycle. The cell debris was cleared out by means of centrifugation. All enzymes were then purified using Akta purifier using His-Trap column FF Crude 5 mL (GE Healthcares. Germany). The buffer was then changed to 50 mM HEPES pH 7.5 or 50 mM TRIS-HCl pH 8.0 using HiPrep desalting column 26/10 50mL (GE Healthcare. Germany). All enzyme was flash frozen in liquid N₂ and prior to storage at -80°C until further use.

Samples were analysed using HPLC (Ultimate-3000 HPLC system (Dionex, Idstein, Germany)), equipped with autosampler and a diode-array detector. Chromatographic separation of gluconate, 2-keto-3-desoxygluconate, pyruvate and glycerate was achieved on a Metrosep A Supp10-250/40 column (250 mm, particle size 4.6 mm; Metrohm,Filderstadt, Germany) at 65 °C by isocratic elution with 12 mm ammonium bicarbonate (pH 10), followed by a washing step with 30 mm sodium carbonate (pH 10.4). Mobile phase flow was adjusted to 0.2 mL min⁻¹. 2,3-dihydroxyisovalerate and 2-ketoisovalerate were separated using a Nucleogel Sugar 810H column (300 mm, 7.8 mm internal diameter; Macherey-Nagel, Düren, Germany) at 60 °C by isocratic elution with 3 mm H₂SO₄ (pH 2.2). Mobile phase flow was adjusted to 0.6 mL min⁻¹. System calibration was performed using external standards of each of the abovementioned intermediates. Samples were prepared by filtration (10 kDa MWCO, modified PES; VWR, Darmstadt, Germany) and diluted.

Additionally, the DNS assay was applied to follow the reaction progress. For the production of aldonic acids and rare sugars it was applied as an easy and fast measurement. At different time points an aliquot of 24 µl was transferred into 96 µl of DNS reagent (10 g 3,5-dinitrosalicylic acid, 300 g sodium potassium tartrate, 16 g sodium hydroxide for 1 liter). Heated up to 95 °C for 5 minutes and afterwards were 100 µl transferred into a flat bottom microtitre plate and measured at 540 nm in a spectrophotometer (Multiskan GO, Thermo Fisher).

### Hydrogen measurement

Hydrogen was measured in the aqueous phase using a NP hydrogen sensor (Unisense, Denmark). The sensor was calibrated using an H₂ saturated solution for 100 % and N₂ saturated solution for 0 %. H₂ was produced by the reaction of 1 M of H₂SO₄ with Zn. The software sensorstrace suite was used for data analysis and monitoring.

For analyzing H₂ in the gas phase, a gas chromatography GC-TCD model Clarus 580 (Perkin Elmer, Germany) was used. Totalchrom (Perkin Elmer) was used to evaluate the data and integrate the chromatograms.

The GC is equipped with a thermal conductivity detector (TCD) for small gases, and Hayesep N column, and a molecular sieve column. Argon is used as the carrier gas. 20 mL of sample were taken from the exhaust, injected into the GC using a glass syringe, and analyzed. For the samples, in the first 10 hours gas was collected directly into a mechanical glass syringe and analyzed via GC-TCD. For the overnight samples, gas samples were taken directly from the gas phase above the reaction, and the amount of H₂ was calculated taking into consideration the total volume of N₂ flowed during this time. The calibration of GC was conducted using air, 100 % H₂, and a predefined commercial gas mixture.

### Biotransformation of D-xylose to D-xylonate

The cell-free bioproduction of D-xylonate was performed in 500 µl scale in Eppendorf tube under aerobic conditions. The solution contained the combination of enzymes as shown in Table 1. 1.0 mM NAD⁺ and 0.1 mM FMN and 400 mM TRIS-HCl pH 8.0. and 100 mM D-xylose at 30 °C. The reaction was carried out in triplicates. The formation of D-xylonate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 1: Enzymes for biotransformation of D-xylose to D-xylonate**

| Enzyme | Protein concentration (mg/ml) |
|---|---|
| HsXyIDHII | 0.3 |
| NmLac | 0.1 |
| ReSH | 1.4 |

**Table 2: Conversion of D-xylose into D-xylonate**

| Time (h) | D-xylose (mM) | D-xylonate (mM) |
|---|---|---|
| 0 | 98.3 | 0.0 |
| 3 | 70.2 | 27.3 |
| 7 | 32.3 | 65.4 |
| 24 | 0.0 | 97.5 |

### Biotransformation of D-glucose to D-gluconate

The cell-free bioproduction of D-gluconate was performed in 500 µl scale in Eppendorf tube under aerobic conditions. The solution contained the combination of enzymes as shown in Table 3. 1.0 mM NAD⁺ and 0.1 mM FMN and 400 mM TRIS-HCl pH 8.0. and 100 mM D-glucose at 30 °C. The reaction was carried out in triplicates. The formation of D-gluconate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 3: Enzyme amounts for biotransformation of D-glucose to D-gluconate**

| Enzyme | Protein concentration (mg/ml) |
|---|---|
| HsXyIDHII or BsGDH | 0.3 |
| NmLac | 0.1 |
| ReSH | 1.4 |

**Table 4: Conversion of D-glucose into D-gluconate**

| Time (h) | D-glucose (mM) | D-gluconate (mM) |
|---|---|---|
| 0 | 101.3 | 0.0 |
| 3 | 73.2 | 27.3 |
| 7 | 29.5 | 65.4 |
| 24 | 0.0 | 98.3 |

### Biotransformation of D-mannose to D-mannonate

The cell-free bioproduction of D-mannonate was performed in 500 µl scale in Eppendorf tube under aerobic conditions. The solution contained the combination of enzymes as shown in Table 5. 1.0 mM NAD⁺ and 0.1 mM FMN and 400 mM TRIS-HCl pH 8.0. and 100 mM D-mannose at 30 °C. The reaction was carried out in triplicates. The formation of D-mannonate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 5: Enzyme amounts for biotransformation of D-mannose to D-mannonate**

| Enzyme | Protein concentration (mg/ml) |
|---|---|
| HsXyIDHII | 0.3 |
| NmLac | 0.1 |
| ReSH | 1.4 |

**Table 6: Conversion of D-mannose into D-mannonate**

| Time (h) | D-mannose (mM) | D-mannonate (mM) |
|---|---|---|
| 0 | 100.9 | 0.0 |
| 2 | 79.2 | 18.7 |
| 6 | 45.3 | 55.9 |
| 24 | 0.0 | 99.1 |

### Biotransformation of D-galactose to D-galactonate

The cell-free bioproduction of D-galactonate was performed in 500 µl scale in Eppendorf tube under aerobic conditions. The solution contained the combination of enzymes as shown in Table 7. 1.0 mM NAD⁺ and 0.1 mM FMN and 400 mM TRIS-HCl pH 8.0. and 100 mM D-galactose at 30 °C. The reaction was carried out in triplicates. The formation of D-galactonate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 7: Enzyme amounts for biotransformation of D-galactose to D-galactonate**

| Enzyme | Protein concentration (mg/ml) |
|---|---|
| HsXyIDHII | 0.3 |
| NmLac | 0.1 |
| ReSH | 1.4 |

**Table 8: Conversion of D-galactose into D-galactonate**

| Time (h) | D-galactose (mM) | D-galactonate (mM) |
|---|---|---|
| 0 | 97.8 | 0.0 |
| 2 | 81.2 | 19.3 |
| 6 | 45.2 | 53.7 |
| 24 | 0.0 | 99.1 |

### Biotransformation of L-arabinose to L-arabinonate

The cell-free bioproduction of L-arabinonate was performed in 500 µl scale in Eppendorf tube under aerobic conditions. The solution contained the combination of enzymes as shown in Table 9. 1.0 mM NAD+ and 0.1 mM FMN and 400 mM TRIS-HCl pH 8.0. and 100 mM L-arabinose at 30 °C. The reaction was carried out in triplicates. The formation of L-arabinonate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 9: Enzyme amounts for biotransformation of L-arabinose to L-arabinonate**

| Enzyme | Protein concentration (mg/ml) |
|---|---|
| HsXyIDHII | 0.3 |
| NmLac | 0.1 |
| ReSH | 1.4 |

**Table 10: Conversion of L-arabinose into L-arabinonate**

| Time (h) | L-arabinose (mM) | L-arabinonate (mM) |
|---|---|---|
| 0 | 103.4 | 0.0 |
| 4 | 62.4 | 35.2 |
| 8 | 22.3 | 79.4 |
| 24 | 0.0 | 97.9 |

### Biotransformation of D-xylose to α-keto-glutarate

The cell-free bioproduction of α-keto-glutarate was performed in 500 µl scale in Eppendorf tube under aerobic conditions. The solution contained the combination of enzymes as shown in Table 11. 1.0 mM NAD⁺, 0.1 mM FMN, 1 mM MgCl₂ and 1000 mM TRIS-HCl pH 8.0. and 100 mM D-xylose at 30 °C. The reaction was carried out in triplicates. The formation of α-keto-glutarate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 11: Enzyme amounts for biotransformation of D-xylose α-keto-glutarate**

| Enzyme | Protein concentration (mg/ml) |
|---|---|
| HsXyIDHII | 0.3 |
| NmLac | 0.1 |
| PuDHT | 0.3 |
| PpD-KdgD | 0.3 |
| PpKgsaDH | 0.3 |
| ReSH | 1.4 |

**Table 12: Conversion of D-xylose into α-keto-glutarate**

| Time (h) | D-xylose (mM) | α-keto-glutarate (mM) |
|---|---|---|
| 0 | 100.4 | 0.0 |
| 2 | 69.3 | 28.3 |
| 8 | 33.4 | 65.4 |
| 24 | 0.0 | 99.1 |

### Biotransformation of L-Arabinose to α-keto-glutarate

The cell-free bioproduction of α-keto-glutarate was performed in 500 µl scale in Eppendorf tube under aerobic conditions. The solution contained the combination of enzymes as shown in Table 13. 1.0 mM NAD⁺, 0.1 mM FMN, 1 mM MgCl₂ and 1000 mM TRIS-HCl pH 8.0. and 100 mM D-xylose at 30 °C. The reaction was carried out in triplicates. The formation of α-keto-glutarate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 13: Enzyme amounts for biotransformation of L-Arabinose to α-keto-glutarate**

| Enzyme | Protein concentration (mg/ml) |
|---|---|
| HsXyIDHII | 0.3 |
| NmLac | 0.1 |
| PuDHT | 0.3 |
| CnL-KdgD | 0.3 |
| PpKgsaDH | 0.3 |
| ReSH | 1.4 |

**Table 14: Conversion of L-arabinose into α-keto-glutarate**

| Time (h) | L-arabinose (mM) | α-keto-glutarate (mM) |
|---|---|---|
| 0 | 102.9 | 0.0 |
| 2 | 67.4 | 27.2 |
| 8 | 26.3 | 73.4 |
| 24 | 0.0 | 100.8 |

### Biotransformation of D-glucuronic acid to α-keto-glutarate

The cell-free bioproduction of α-keto-glutarate was performed in 500 µl scale in Eppendorf tube under aerobic conditions. The solution contained the combination of enzymes as shown in Table 15. 1.0 mM NAD⁺, 0.1 mM FMN, 1 mM MgCl₂ and 1000 mM TRIS-HCl pH 8.0. and 100 mM D-glucuronic acid at 30 °C. The reaction was carried out in triplicates. The formation of α-keto-glutarate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 15: Enzyme amounts for biotransformation of D-glucuronic acid to α-keto-glutarate**

| Enzyme | Protein concentration (mg/ml) |
|---|---|
| AtUDH | 0.2 |
| AsGlucD | 0.2 |
| AbKdgD | 0.3 |
| PpKgsaDH | 0.3 |
| ReSH | 1.4 |

**Table 16: Conversion of D-glucuronic acid to α-keto-glutarate**

| Time (h) | D-glucuronic acid (mM) | α-keto-glutarate (mM) |
|---|---|---|
| 0 | 99.1 | 0.0 |
| 2 | 71.3 | 27.4 |
| 8 | 35.1 | 68.2 |
| 24 | 0.0 | 98.3 |

### Biotransformation of D-galacturonic acid to α-keto-glutarate

The cell-free bioproduction of α-keto-glutarate was performed in 500 µl scale in Eppendorf tube under aerobic conditions. The solution contained the combination of enzymes as shown in Table 17. 1.0 mM NAD⁺, 0.1 mM FMN, 1 mM MgCl₂ and 1000 mM TRIS-HCl pH 8.0. and 100 mM D-galacturonic acid at 30 °C. The reaction was carried out in triplicates. The formation of α-keto-glutarate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 17: Enzyme amounts for biotransformation of D-galacturonic acid to α-keto-glutarate**

| Enzyme | Protein concentration (mg/ml) |
|---|---|
| AtUDH | 0.2 |
| AsGlucD | 0.3 |
| AbKdgD | 0.3 |
| PpKgsaDH | 0.3 |
| ReSH | 1.4 |

**Table 18: Conversion of D-galacturonic acid to α-keto-glutarate**

| Time (h) | D-glucuronic acid (mM) | α-keto-glutarate (mM) |
|---|---|---|
| 0 | 101.7 | 0.0 |
| 2 | 71.3 | 27.4 |
| 8 | 35.1 | 68.2 |
| 24 | 0.0 | 100.2 |

### Biotransformation of D-glucose to α-keto-glutarate

The cell-free bioproduction of α-keto-glutarate was performed in 500 µl scale in Eppendorf tube under aerobic conditions. The solution contained the combination of enzymes as shown in Table 19. 1.0 mM NAD⁺, 0.1 mM FMN, 1 mM MgCl₂ and 1000 mM TRIS-HCl pH 8.0. and 100 mM D-glucose at 30 °C. The reaction was carried out in triplicates. The formation of α-keto-glutarate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min. Hydrogen was detected in the aqueous phase using a NP hydrogen sensor (Unisense, Denmark).

**Table 19: Enzyme amounts for biotransformation of D-glucose to α-keto-glutarate**

| Enzyme | Protein concentration (mg/ml) |
|---|---|
| BsGDH | 0.3 |
| SspADH | 3.0 |
| AtUDH | 1.5 |
| AsGlucD | 0.2 |
| AbKdgD | 0.3 |
| PpKgsaDH | 0.3 |
| ReSH | 1.4 |

**Table 20: Conversion of D-glucose into α-keto-glutarate**

| Time (h) | D-glucose (mM) | α-keto-glutarate (mM) |
|---|---|---|
| 0 | 99.2 | 0.0 |
| 2 | 88.2 | 10.4 |
| 8 | 73.4 | 25.9 |
| 24 | 15.4 | 83.9 |
| 48 | 0.0 | 99.7 |

### Biotransformation of D-xylose to succinate

The cell-free bioproduction of succinate was performed in 500 µl scale in Eppendorf tube under aerobic conditions. The solution contained the combination of enzymes as shown in Table 21. 1.0 mM NAD⁺, 0.1 mM FMN, 5 mM MgCl₂ and 1000 mM TRIS-HCl pH 8.0. and 100 mM D-xylose at 30 °C. The reaction was carried out in triplicates. The formation of succinate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 21: Enzyme amounts for biotransformation of D-xylose to succinate**

| Enzyme | Protein concentration (mg/ml) |
|---|---|
| HsXylDHII | 0.3 |
| NmLac | 0.1 |
| PuDHT | 0.3 |
| PpD-KdgD | 0.3 |
| PpKgsaDH | 0.3 |
| MtKgd | 1.0 |
| ReSH | 1.4 |

**Table 22: Conversion of D-xylose into succinate**

| Time (h) | D-xylose (mM) | succinate (mM) |
|---|---|---|
| 0 | 101.3 | 0.0 |
| 3 | 75.6 | 22.9 |
| 6 | 52.8 | 46.9 |
| 24 | 0.0 | 100.4 |

### Biotransformation of L-arabinose to succinate

The cell-free bioproduction of succinate was performed in 500 µl scale in Eppendorf tube under aerobic conditions. The solution contained the combination of enzymes as shown in Table 23. 1.0 mM NADH, 0.1 mM FMN, 1 mM MgCl₂ and 1000 mM TRIS-HCl pH 8.0. and 100 mM L-arabinose at 30 °C. The reaction was carried out in triplicates. The formation of succinate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min. Hydrogen was detected in the aqueous phase using a NP hydrogen sensor (Unisense, Denmark).

**Table 23: Enzyme amounts for biotransformation of L-arabinose to succinate**

| Enzyme | Protein concentration (mg/ml) |
|---|---|
| HsXylDHII | 0.3 |
| NmLac | 0.1 |
| PuDHT | 0.3 |
| CnL-KdgD | 0.3 |
| PpKgsaDH | 0.3 |
| MtKgd | 1.0 |
| ReSH | 1.4 |

**Table 24: Conversion of L-arabinose into succinate**

| Time (h) | L-arabinose (mM) | succinate (mM) |
|---|---|---|
| 0 | 98.9 | 0.0 |
| 3 | 77.2 | 23.4 |
| 6 | 56.1 | 44.2 |
| 24 | 0.0 | 99.7 |

### Biotransformation of D-xylose to 3,4-dihydroxy-butanoate

The cell-free bioproduction of 3,4-dihydroxy-butanoate was performed in 500 µl scale in Eppendorf tube under aerobic conditions. The solution contained the combination of enzymes as shown in Table 25. 1.0 mM NAD⁺, 0.1 mM FMN, 0.1 mM TPP, 5 mM MgCl₂ and 1000 mM TRIS-HCl pH 8.0. and 100 mM D-xylose at 30 °C. The reaction was carried out in triplicates. The formation of 3,4-dihydroxy-butanoate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 25: Enzyme amounts for biotransformation of D-xylose to 3,4-dihydroxy-butanoate**

| Enzyme | Protein concentration (mg/ml) |
|---|---|
| HsXylDHII | 0.3 |
| NmLac | 0.1 |
| PuDHT | 0.3 |
| LlKdcA | 1.0 |
| VpALDH | 0.3 |
| ReSH | 1.4 |

**Table 26: Conversion of D-xylose into 3,4-dihydroxy-butanoate**

| Time (h) | D-xylose (mM) | 3,4-dihydroxy-butanoate (mM) |
|---|---|---|
| 0 | 101.3 | 0.0 |
| 2 | 75.6 | 22.9 |
| 7 | 52.8 | 46.9 |
| 24 | 0.0 | 100.4 |

### Biotransformation of L-arabinose to 3,4-dihydroxy-butanoate

The cell-free bioproduction of 3,4-dihydroxy-butanoate was performed in 500 µl scale in Eppendorf tube under aerobic conditions. The solution contained the combination of enzymes as shown in Table 27. 1.0 mM NAD⁺, 0.1 mM FMN, 5 mM MgCl₂ and 1000 mM TRIS-HCl pH 8.0. and 100 mM L-arabinose at 30 °C. The reaction was carried out in triplicates. The formation of 3,4-dihydroxy-butanoate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 27: Enzyme amounts for biotransformation of L-arabinose to 3,4-dihydroxy-butanoate**

| Enzyme | Protein concentration (mg/ml) |
|---|---|
| HsXyIDHII | 0.3 |
| NmLac | 0.1 |
| PuDHT | 0.3 |
| LIKdcA | 1.0 |
| VpALDH | 0.3 |
| ReSH | 1.4 |

**Table 28: Conversion of L-arabinose into 3,4-dihydroxy-butanoate**

| Time (h) | L-arabinose (mM) | 3,4-dihydroxy-butanoate (mM) |
|---|---|---|
| 0 | 102.1 | 0.0 |
| 3 | 71.9 | 28.4 |
| 7 | 57.1 | 42.8 |
| 24 | 0.5 | 95.1 |

### Biotransformation of D-glucose to pyruvate

The cell-free bioproduction of pyruvate was performed in 500 µl scale in Eppendorf tube. The solution contained the combination of enzymes as shown in Table 29. 1.0 mM NAD⁺, 0.1 mM FMN, 5 mM MgCl₂. and 1000 mM TRIS-HCl and 100 mM D-glucose. The reaction was carried out in triplicates. The formation of pyruvate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 29: Enzyme amounts for biotransformation of D-glucose to pyruvate**

| Enzyme | Protein concentration (mg/ml) |
|---|---|
| BsGDH | 0.3 |
| NmLac | 0.1 |
| PuDHT | 1.0 |
| PtKdgA | 0.3 |
| VpALDH | 0.3 |
| ReSH | 1.4 |

**Table 30: Conversion of D-glucose into pyruvate**

| Time (h) | D-glucose (mM) | pyruvate (mM) |
|---|---|---|
| 0 | 102.7 | 0.0 |
| 3 | 65.7 | 71.1 |
| 8 | 48.1 | 105.2 |
| 24 | 0.0 | 197.2 |

### Biotransformation of D-glucose to acetic acid

The cell-free bioproduction of acetic acid was performed in 500 µl scale in Eppendorf tube. The solution contained the combination of enzymes as shown in Table 29. 1.0 mM NAD⁺, 0.1 mM FMN, 0.1 TPP, 5 mM MgCl₂. and 1000 mM TRIS-HCl and 100 mM D-glucose. The reaction was carried out in triplicates. The formation of pyruvate was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 31: Enzyme amounts for biotransformation of D-glucose to acetic acid**

| Enzyme | Protein concentration (mg/ml) |
|---|---|
| BsGDH | 0.3 |
| NmLac | 0.1 |
| PuDHT | 1.0 |
| PtKdgA | 0.3 |
| VpALDH | 0.3 |
| ApPDC | 0.3 |
| ReSH | 1.4 |

**Table 32: Conversion of D-glucose into acetic acid**

| Time (h) | D-glucose (mM) | pyruvate (mM) |
|---|---|---|
| 0 | 102.7 | 0.0 |
| 3 | 85.4 | 33.5 |
| 8 | 65.1 | 69.2 |
| 24 | 47.3 | 100.8 |
| 48 | 13.7 | 164.8 |

### Biotransformation of HMF to FDCA

The cell-free bioproduction of FDCA was performed in 500 µl scale in Eppendorf tube. The solution contained the combination of enzymes as shown in Table 31. 1.0 mM NAD⁺, 0.1 mM FMN, 5 mM MgCl₂. and 1000 mM TRIS-HCl and 50 mM HMF. The reaction was carried out in triplicates. The formation of FDCA was followed over time by withdrawing 20 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.
Hydrogen was detected in the aqueous phase using a NP hydrogen sensor (Unisense, Denmark).

**Table 33: Enzyme amounts for biotransformation of HMF to FDCA**

| Enzyme | Protein concentration (mg/ml) |
|---|---|
| EcYjgB-LND | 0.5 |
| VpALDH | 0.5 |
| ReSH | 1.4 |

**Table 34: Conversion of HMF into FDCA**

| Time (h) | HMF (mM) | FDCA (mM) |
|---|---|---|
| 0 | 49.2 | 0.0 |
| 3 | 40.3 | 8.9 |
| 7 | 31.2 | 20.3 |
| 24 | 0.0 | 48.7 |

### Biotransformation of D-sorbitol to L-gulose

The cell-free bioproduction of L-gulose was performed in 500 µl scale in Eppendorf tube. The solution contained the combination of enzymes as shown in Table 33. 1.0 mM NAD⁺, 0.1 mM FMN, 1 mM MgCl₂. and 100 mM TRIS-HCl pH 8.0. and 100 mM D-sorbitol. The reaction was carried out in triplicates. The formation of L-gulose was followed over time by withdrawing 20 µl of aliquot at certain time intervals. The reaction was followed with a HPLC Dionex Ultimate 3000 system equipped with autosampler (WPS 3000TRS), a column compartment (TCC3000RS) and a light scattering detector. The YMC Triart Diol Hilic column (100 * 2 mm, 1.9 µm, 12 nm) at 7 °C was used for separation by gradient elution (15-85%) with 0.1% formic acid pH 4.5, and 0.1% formic acid in ACN as the mobile phase at 0.45 ml min-1. Samples were diluted in water/acetonitrile with the ratio of 3:7. Data were analyzed using Dionex Chromeleon software. Hydrogen was detected in the aqueous phase using a NP hydrogen sensor (Unisense, Denmark).

**Table 35: Enzyme amounts for biotransformation of D-sorbitol to L-gulose**

| Enzyme | Protein concentration (mg/ml) |
|---|---|
| SspADH | 4.0 |
| ReSH | 1.4 |

**Table 36: Conversion of D-sorbitol into L-gulose**

| Time (h) | D-sorbitol (mM) | L-gulose (mM) |
|---|---|---|
| 0 | 98.7 | 0.0 |
| 3 | 90.3 | 8.9 |
| 7 | 78.6 | 21.1 |
| 24 | 45.6 | 54.7 |
| 48 | 25.1 | 72.9 |

### Biotransformation of Dulcitol to L-tagatose

The cell-free bioproduction of L-tagatose was performed in 500 µl scale in Eppendorf tube. The solution contained the combination of enzymes as shown in Table 33. 1.0 mM NAD⁺, 0.1 mM FMN, 1 mM MgCl₂. and 100 mM TRIS-HCl pH 8.0. and 100 mM dulcitol. The reaction was carried out in triplicates. The formation of L-tagatose was followed over time by withdrawing 20 µl of aliquot at certain time intervals. The reaction was followed with a HPLC Dionex Ultimate 3000 system equipped with autosampler (WPS 3000TRS), a column compartment (TCC3000RS) and a light scattering detector. The YMC Triart Diol Hilic column (100 * 2 mm, 1.9 µm, 12 nm) at 7 °C was used for separation by gradient elution (15-85%) with 0.1% formic acid pH 4.5, and 0.1% formic acid in ACN as the mobile phase at 0.45 ml min-1. Samples were diluted in water/acetonitrile with the ratio of 3:7. Data were analyzed using Dionex Chromeleon software. Hydrogen was detected in the aqueous phase using a NP hydrogen sensor (Unisense, Denmark).

**Table 35: Enzyme amounts for biotransformation of dulcitol to L-tagatose**

| Enzyme | Protein concentration (mg/ml) |
|---|---|
| RsGatDH | 1.0 |
| ReSH | 1.4 |

**Table 36: Conversion of dulcitol into L-tagatose**

| Time (h) | dulcitol (mM) | L-tagatose (mM) |
|---|---|---|
| 0 | 99.3 | 0.0 |
| 3 | 85.4 | 13.9 |
| 7 | 69.7 | 30.8 |
| 24 | 45.2 | 53.7 |
| 48 | 23.8 | 73.9 |

Above biotransformations show that the respective organic substances comprising at least one oxygen containing functional group could be converted to the desired products, that is, organic substances comprising at least one carbonyl group. The products were even reacted further in an enzymatic cascade. The experiments proof that NADH can be efficiently converted into NAD+, that is, it is recycled. Furthermore, it has been shown that hydrogen is generated which could be detected during the transformation processes. Hydrogen detection was done in the aqueous reaction solution as well as in the gas phase to proof activity of the hydrogenase.

## Claims

1. Process for preparing an organic substance comprising at least one carbonyl group comprising the steps of
converting an organic substance comprising at least one oxygen-containing functional group into the organic substance comprising at least one carbonyl group and NAD+ and/or NADP+ into NADH and/or NADPH using a dehydrogenase, and
converting NADH and/or NADPH into NAD+ and/or NADP+ and hydrogen using a hydrogenase.

2. Process according to claim 1,
wherein the oxygen-containing functional group is selected from the group of functional groups consisting of hydroxyl, ketone, aldehyde, ester, amid, ether, semi-acetal or acetal, preferably is a hydroxyl group, an aldehyde group or a ketone group, more preferably is a hydroxyl group or aldehyde group; and/or
wherein the at least one carbonyl group is selected from the group consisting of aldehyde group, ketone group or carboxyl group.

3. Process according to any of the preceding claims,
wherein the process is a one-pot reaction; and/or
wherein the process is 100% atom efficient; and/or
wherein the process is run at a temperature in the range of from 5 to 100°C, preferably of from 15 to 60°C, and more preferably of from 20° to 40°C.

4. Process according to any of the preceding claims,
wherein NAD+/NADH and/or NADP+/NADPH is applied in non-stochiometric quantities; and/or
wherein the yield of the conversion from NADH to NAD+ is more than 75%, preferably more than 80% and more preferably more than 90%; and/or
wherein no sacrificial substrate is necessary, preferably no sacrificial substrate for NAD+/NADH and/or NADP+/NADPH recycling is necessary; and/or
wherein the process does not need the addition of O₂, preferably does not need the addition of O₂ for NAD+/NADH and/or NADP+/NADPH recycling.

5. Process according to any of the preceding claims,
wherein the oxidized substance comprising at least one carbonyl group is converted further using further enzymes; and/or
wherein at least one further enzyme is used.

6. Process according to any of the preceding claims,
wherein the hydrogenase is selected from the group consisting of Ralstonia eutropha (SH) and/or SHE341A/S342R, preferably is Ralstonia eutropha (SH); and/or
wherein an oxidoreductase is not applied in the process.

7. Process according to any of the preceding claims,
wherein the conversion of the organic substrate comprising at least one oxygen-containing functional group to obtain the organic substance comprising at least one carbonyl group is part of a multi-enzymatic cascade.

8. Process according to any of the preceding claims,
wherein the organic substance comprising at least one oxygen-containing functional group is a carbohydrate; or
wherein the organic substance comprising at least one oxygen-containing functional group, preferably at least one hydroxyl group or aldehyde group, comprises at least one additional oxygen-containing functional group, preferably at least one additional hydroxyl group; and/or
wherein the organic substance comprising at least one oxygen-containing functional group is a polyol.

9. Process according to any of the preceding claims,
wherein the organic substance comprising at least one oxygen-containing functional group is an aldose, preferably the aldose is converted to an aldonic acid or a salt thereof; or
wherein the organic substance comprising at least one oxygen-containing functional group is a C6 or C5 carbohydrate being converted to α-keto-glutarate; or
wherein the organic substance comprising at least one oxygen-containing functional group is a C6 or C5 carbohydrate being converted to succinic acid or a salt thereof; or
wherein the organic substance comprising at least one oxygen-containing functional group is a C6 carbohydrate being converted to pyruvate; or
wherein the organic substance comprising at least one oxygen-containing functional group is a polyol being converted to an aldose or
wherein the organic substance comprising at least one oxygen-containing functional group is a furfural or hydroxymethyl furfural being converted to a carboxylic acid or dicarboxylic acid.

10. Process according to any of the preceding claims,
wherein the hydrogen generated during the process is removed from the process; and/or
wherein the process is not sensitive to O₂; and/or
wherein the process is a cell-free process.

11. Process according to any of the preceding claims,
wherein the process is a circular process; and/or
wherein the process is a continuous process; and/or
wherein the NAD+/NADH and/or NADP+/NADPH is recycled in the process.

12. Organic substance comprising at least one carbonyl group obtainable by the process according to claims 1 to 11.

13. Use of a hydrogenase for recycling NADH to NAD+ and/or NADPH to NADP+ in a process applying a dehydrogenase and an organic substance with at least one oxygen-containing functional group.

14. Use of a hydrogenase for generating hydrogen in a process using a dehydrogenase, an organic substance comprising at least one oxygen-containing functional group and NAD+/NADH and/or NADP+/NADPH.

15. Use of a hydrogenase in a process for preparing an organic substance comprising at least one carbonyl group applying a dehydrogenase and NADH and/or NADPH, for generating hydrogen and NAD+ and/or NADP+.
